# EUROPÄISCHE PATENTSCHRIFT

(11) **EP 0 723 763 B1**
(45) Veröffentlichungstag und Bekanntmachung des Hinweises auf die Patenterteilung: **26.05.1999**
(21) Anmeldenummer: 96100001.5
(22) Anmeldetag: 02.01.1996
(51) Int. Cl.: A61B 17/34, A61B 19/00, A61B 6/08

(54) **Zielvorrichtung für das geradlinige Einführen eines Instruments in einen menschlichen Körper**
Aiming device for linearly introducing an instrument into a human body
Dispositif de visée pour introduire en ligne droite un instrument dans le corps humain

(30) Priorität: 26.01.1995 DE 19502356
(43) Veröffentlichungstag der Anmeldung: 31.07.1996
(73) Patentinhaber: LAP GmbH Laser Applikationen, D-21337 Lüneburg (DE)
(72) Erfinder: Röckseisen, Armin, Dr., D-21379 Scharnebeck (DE)
(74) Vertreter: Patentanwälte Hauck, Graalfs, Wehnert, Döring, Siemons

(56) Entgegenhaltungen:
- WO-A-88/08282
- FR-A- 2 284 338
- US-A- 4 246 486
- US-A- 4 629 989
- US-A- 4 651 732
- US-A- 5 100 411
- US-A- 5 320 111

## Beschreibung

Die Erfindung bezieht sich auf eine Zielvorrichtung für das geradlinige Einführen eines Instruments in einen menschlichen Körper nach dem Oberbegriff des Patentanspruchs 1.

So eine Zielvorrichtung ist aus US-A-4651732 bekannt.

Bei der Biopsie werden Nadeln in den Körper des Patienten gestochen, um aus einer bestimmten Körperstelle Gewebsproben zu entnehmen oder Instrumente zu dieser Stelle zu führen. Es ist bekannt, die Zielstelle mit Hilfe eines Computertomographen (CT) zu bestimmen. Er erzeugt ein virtuelles Schnittbild des Körpers in verschiedenen Schnittebenen. Nach dem CT-Bild kann die Zielstelle ermittelt werden und die Einstichstelle und der Verlauf des Stichkanals bestimmt werden. Wenn der Einstich nicht senkrecht von oben oder waagerecht von der Seite erfolgen kann, weil Organe oder Knochen im Wege sind, ist es schwierig, den durch das CT-Bild ermittelten Einstichwinkel in die Realität zu übertragen und die lange Nadel im richtigen Winkel zu führen. Findet eine Abweichung vom richtigen Winkel statt, wird die Zielstelle verpaßt und die Prozedur muß wiederholt werden.

Der Erfindung liegt daher die Aufgabe zugrunde, eine Zielvorrichtung für das geradlinige Einführen eines Instruments in einen menschlichen Körper zu schaffen, mit der auf einfache Weise das Instrument im vorgegebenen Winkel zur vorbestimmten Zielstelle geführt werden kann.

Diese Aufgabe wird durch die Merkmale des Patentanspruchs 1 gelöst.

Erfindungsgemäß ist ein Schlitten oberhalb der Gantry (Einführöffnung) des Computertomographen entlang einer horizontalen Führung, vorzugsweise entlang einer Transversalebene, verstellbar geführt und an einem beliebigen Punkt feststellbar. Der Schlitten lagert eine Laservorrichtung um eine horizontale Achse. Die Laservorrichtung erzeugt eine Zielmarkierung, zum Beispiel in Form eines Punktes oder eines Kreuzes im Raum. Bei der Verschwenkung der Laservorrichtung wird der Laserstrahl in einer vertikalen Ebene entsprechend mit verschwenkt. Am Schlitten ist außerdem eine Winkelskala angebracht, an der der Schwenkwinkel der Laservorrichtung abgelesen werden kann. Es versteht sich, daß auch bei einem beliebigen Schwenkwinkel die Laservorrichtung vorzugsweise arretiert werden kann.

Die Laservorrichtung ist vorzugsweise eine Einheit zum projizieren eines Linienkreuzes.

Alternativ kann ein Kreuz auch dadurch projiziert werden, daß ein Linienlaser raumfest angebracht, dessen Strahlebene in einer Transversalebene bezüglich des Patientenkörpers verläuft. Ein zweiter Linienlaser, dessen Strahlebene in der Nullstellung in der Sagittalebene verläuft, ist am Schlitten schwenkbar angebracht.

Die Führung ist am Gehäuse des Computertomographen oberhalb der Gantry angebracht, beispielsweise in Form einer horizontalen Schiene. Die Schiene kann mit senkrecht dazu verlaufenden Armen versehen sein, die mit länglichen Halteprofilen beidseits der Gantry zusammenwirken.

Der Patient wird auf einer entlang einer Führung verfahrbaren Liege in den Computertomographen hinein und aus diesem herausbewegt. Beim Start befindet sich der die Laservorrichtung lagernde Verfahrschlitten in der Mitte der Führung bzw. der Schiene und die Laservorrichtung ist in Nullstellung senkrecht nach unten gerichtet, wobei in dieser Position z.B. ein Kreuz senkrecht von oben auf den Patienten projiziert wird. Alternativ kann auch ein Punkt projiziert werden. Die Sagittallinie verläuft bekanntlich in der Mitte der Liege und des CT und die Transversallinie der Laservorrichtung verläuft parallel zur Scanebene des CT in einem festen aber bekannten Abstand. Nach diesen Linien kann der Patient auf der Liege ausgerichtet werden. Der Patient wird auf der Linie mit der zu scannenden Körperpartie in die Transversalebene gefahren und markiert. Diese Markierung dient dem äußerlich wiedererkennbaren Bezug von Scanebene und Patientenkörper. Dann wird der Patient gescannt. Der Arzt wählt aus den CT-Bildern die geeignete Scanebene für die Intervention aus. Unter Berücksichtigung des erwähnten Abstands wird der Patient mit der Liege so weit herausgefahren, bis die gewählte Scanebene in der Ebene der Transversallinie liegt.

Der Arzt bestimmt die Einstichstelle und den Einstichwinkel aus den CT-Bildern. Er legt die seitliche Lage der Einstichstelle nach markanten anatomischen Merkmalen des Patienten fest und markiert die Einstichstelle. Anschließend schwenkt er die Laservorrichtung in die festgelegte Winkelstellung, indem er zum Beispiel eine Marke an der stabförmigen Laservorrichtung auf den gewählten Winkel bringt und anschließend die Laservorrichtung arretiert. Anschließend wird der Verfahrschlitten mit dem geschwenkten Laser so lange seitwärts gefahren, bis das Laserkreuz die Markierung der Einstichstelle trifft. Das Instrument, zum Beispiel eine Nadel, muß so auf die Einstichstelle gesetzt werden, daß ihre Rückseite vom Laserkreuz getroffen wird. Ist das der Fall, ist die Nadel im vorher festgelegten Winkel auf die Zielstelle ausgerichtet.

Es versteht sich, daß auch drei Systeme der beschriebenen Art verwendet werden können, bei denen eine Führung horizontal und zwei senkrecht seitlich der Gantry angebracht sind, um Schwenkwinkel größer als 45° zu verwirklichen.

Die Erfindung wird nachfolgend anhand von Zeichnungen näher erläutert.
- Fig. 1: zeigt schematisch die Frontansicht eines Computertomographen mit einer Vorrichtung nach der Erfindung.
- Fig. 2: zeigt eine Einzelheit der Vorrichtung nach Fig. 1.
- Fig. 3: zeigt detailliert ein Teil der Vorrichtung nach Fig. 1.
- Fig. 4: zeigt eine Einzelheit der Vorrichtung nach Fig. 2.

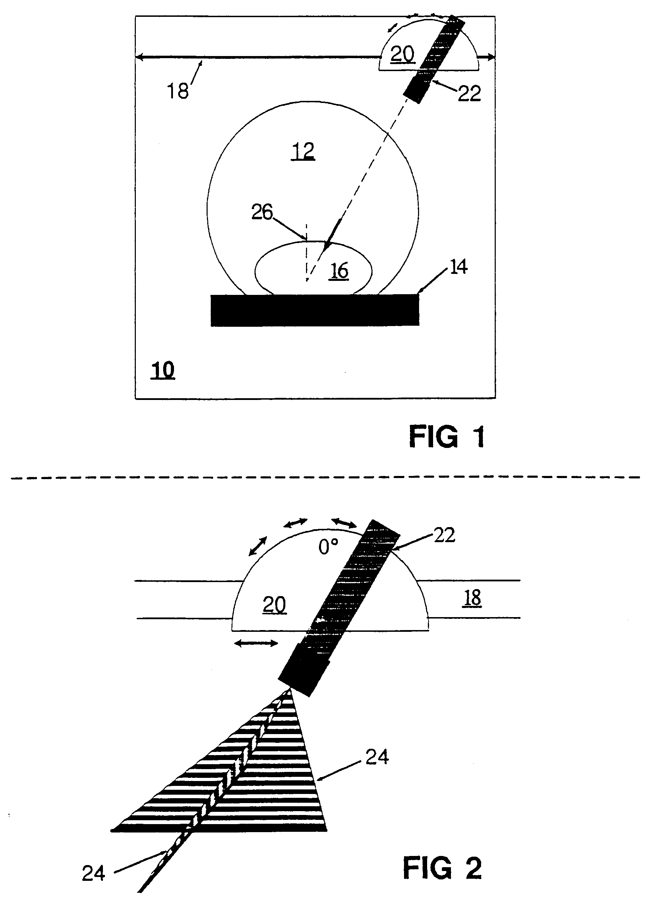

In Fig. 1 ist die Frontseite eines Computertomographen dargestellt mit einem Gehäuse 10 und einer Gantry 12. Man erkennt außerdem eine Liege 14, auf der ein Patient 16 angedeutet liegt. Die Liege 14 ist linear verfahrbar in den Computertomographen hinein. Oberhalb der Gantry 12 ist eine horizontale Führung 18 angeordnet, an der ein Schlitten 20 verschiebbar ist. Der Schlitten 20 kann in einer beliebigen Stellung an der Führung 18 arretiert werden. Der Schlitten 20 lagert schwenkbar eine stabförmige Laservorrichtung 22, die zum Beispiel ein Kreuz projiziert. Zu diesem Zweck erzeugt die Laservorrichtung 22 zwei senkrecht zueinander stehende Linien, von denen die eine in einer Transversalebene, die in Fig. 2 mit 24 bezeichnet ist und die andere in einer Sagittalebene, die in Fig. 2 mit 24 bezeichnet ist, verläuft. Wie aus Fig. 1 zu erkennen, erstrecken sich beide genannten Strahlungsebenen in einem Winkel zur Sagittalebene des Patienten, die in Fig. 1 mit 26 beschrieben ist.

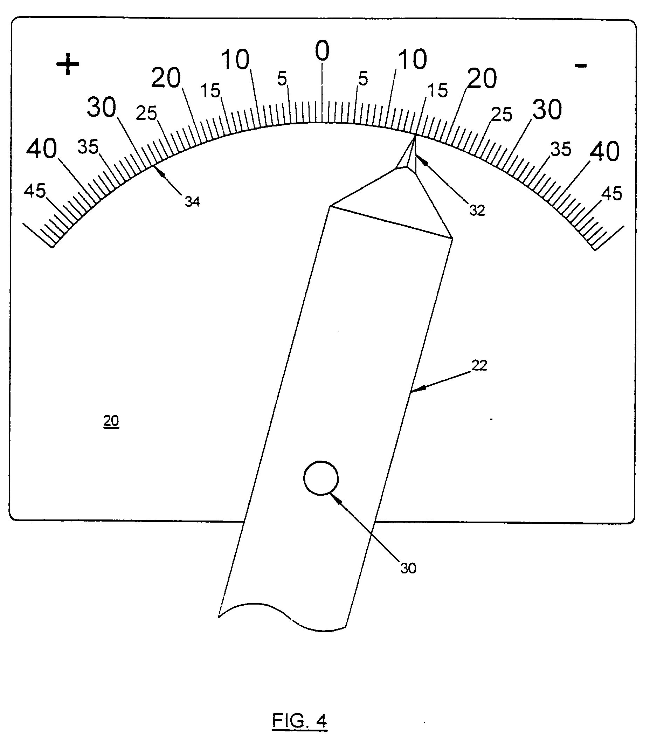

In Fig. 4 ist ein alternativer Schlitten 20' dargestellt mit einer Laservorrichtung 22', die bei 30 um eine horizontale Achse am Schlitten 20' schwenkbar gelagert ist. In beliebiger Winkelstellung kann jedoch eine Arretierung der Laservorrichtung 22 vorgenommen werden. An der Laservorrichtung 22' ist eine Spitze am hinteren Ende 32 vorgesehen, die mit einer Skala 34 am Schlitten 20' zusammenwirkt. An der Skala ist die Größe der Winkelstellung der Laservorrichtung 22' ablesbar.

Wie schon erwähnt, wird die Einführstelle zum Beispiel einer Biopsienadel am Patientenkörper mit Hilfe des CT ermittelt und vom Arzt am Körper markiert. Dem Arzt ist ferner durch die CT-Bilder der Winkel bekannt, in dem das Instrument gehalten bzw. geführt werden muß, um ohne Beeinträchtigung von Knochen oder Organen das Instrument zur Zielstelle des Patienten zu führen. Die Winkelstellung stellt der Arzt dadurch ein, daß er die Laservorrichtung 22 bzw. 22' auf den vorgegebenen Winkel einstellt und arretiert. Zuvor war der Patient so weit aus dem Computertomographen herausgefahren, bis die am Körper vorgenommene Markierung mit der Transversalebene 24 der Laservorrichtung 22 zusammenfällt. Anschließend wird der Schlitten 20 bzw. 20' so weit entlang der Führung 18 verfahren, bis auch die Sagittalebene 24 durch die Markierung geht. Nunmehr ist das Instrument so zu halten und zu führen, daß am hinteren Ende das Kreuz der Laservorrichtung 22 bzw. 22' projiziert wird.

In Fig. 4 ist angedeutet, wie die gezeigte Zielvorrichtung nach den Figuren 1 bis 3 mechanisch verwirklicht werden kann. Die Führung 18 wird von einer Schiene 40 gebildet, an der ein Schlitten 42 in geeigneter Weise präzise geführt wird. Auf diese Führung wird im einzelnen nicht eingegangen. An den Enden der Schiene 40 sind senkrecht dazu abstehend Arme 44, 46 angebracht, die an der Außenseite Rastbolzen 48 bzw. 50 aufweisen. Die Rastbolzen sind von Federn beaufschlagt, wodurch sie in die Ursprungsstellung zurückgebracht werden, wenn sie von Hand herausgezogen werden.

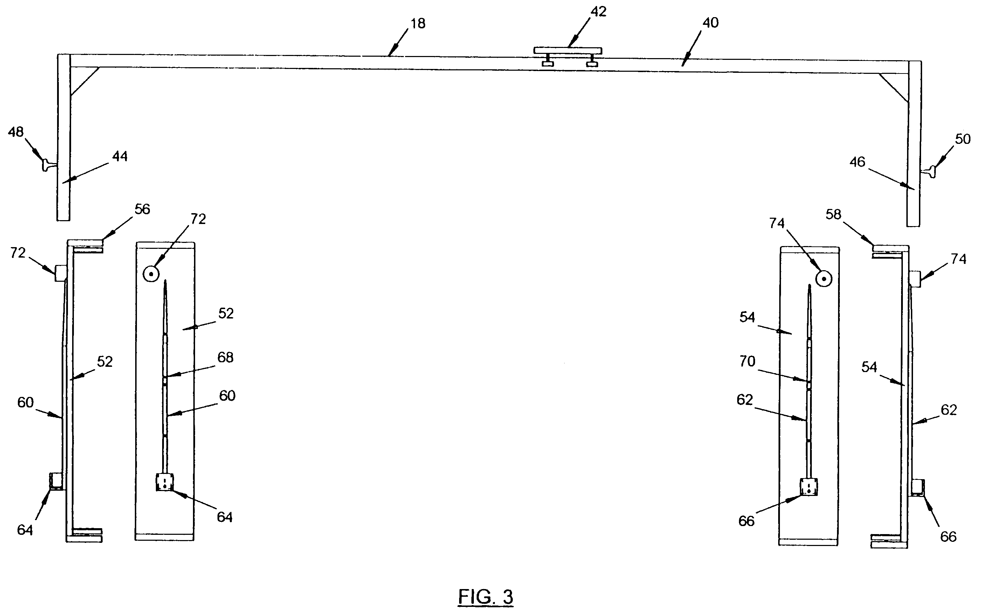

Beidseits der Gantry 12 ist am Gehäuse 10 eine Halteplatte 52 bzw. 54 angebracht. In Fig. 3 ist die Halteplatte 52 bzw. 54 einmal in Seitenansicht (wie am Gehäuse des CT befestigt) und einmal in Frontansicht gezeigt. Jede Platte 52 bzw. 54 ist mit zwei Klemmvorrichtung 56 bzw. 58 versehen zur Anbringung am Gehäuse mit einer entsprechenden Einstellung der Parallelität. An der Außenseite der Platte 52, 54 ist eine Führungsleiste 60 bzw. 62 angeordnet, die im oberen Bereich rampenartig ausgebildet ist und sowohl in der Höhe allmählich ansteigt als auch in der Breite allmählich größer wird. Dieser rampenartige Abschnitt dient zum Einführen in das nicht gezeigte komplementäre Profil der Arme 44, 46. An der Außenseite im unteren Bereich ist ferner eine Aufnahme 64 bzw. 66 für die Arme 48, 50 vorgesehen. In der Leiste 60, 62 befindet sich eine Bohrung 68 bzw. 70 für den Rastbolzen 48, 50. Dadurch können die Arme 44, 46 lagerichtig positioniert werden. Dadurch erhält auch die Führung 18 eine präzise Lage im Raum. Schließlich ist im oberen Bereich seitlich der Leisten 60, 62 eine Rolle 72, 74 drehbar gelagert. Sie dient zum erleichterten Einführen des Profils der Arme 44, 46 in die Halterung.

## Patentansprüche

1. Zielvorrichtung zum geradlinigen Einführen eines länglichen Instruments in einen menschlichen Körper an eine mit Hilfe eines Computertomographen ermittelte und durch einen Auftrag markierte Zielstelle mit
- einem eine Gantry (12) aufweisenden Computertomographen (CT) zum Erzeugen von transversalen Schnittbildern des Körpers in einer transversalen Scan-Ebene,
- einer entlang einer Führung horizontal in Längsrichtung der Gantry verfahrbaren Liege (14) zur Lagerung des Patienten,
dadurch gekennzeichnet, daß
- die Zielvorrichtung zum geradlinigen Einführen eines länglichen Instruments in einer bestimmten transversalen Schittebene dient, und daß die Zielvorrichtung
- eine oberhalb der Gantry (12) am CT angebrachten horizontalen Führung (18) mit einem entlang der horizontalen Führung verfahrbaren Schlitten (20) mit Arretiermitteln zum Arretieren des Schlittens (20) an einem beliebigen Punkt der horizontalen Führung (18),
- eine Laservorrichtung (22, 22') zum Projizieren einer Zielmarkierung,
- eine Lagervorrichtung, die die Laservorrichtung (22, 22') um eine horizontale Achse schwenkbar lagert, derart daß der Laserstrahl in einer Transversalebene schwenkbar ist, die sich im vorgegebenen Abstand zur transversalen Scan-Ebene befindet, und
- eine Winkelskala (34) am Schlitten (20) zur Anzeige des Schwenkwinkels der Laservorrichtung (22, 22') aufweist.

2. Vorrichtung nach Anspruch 1, dadurch gekennzeichnet, daß die Laservorrichtung (22) als Einheit zum Projizieren eines Linienkreuzes ausgebildet ist.

3. Vorrichtung nach Anspruch 1, dadurch gekennzeichnet, daß die Laservorrichtung zum Projizieren eines Kreuzes ausgebildet ist, wobei ein Linienlaser raumfest angebracht ist und seine Strahlebene in der Transversalebene des Körpers verläuft, die sich in bestimmtem Abstand von der Scan-Ebene des CT befindet, und ein zweiter Linienlaser am Schlitten um die horizontale Achse schwenkbar gelagert ist und seine Strahlebene in der Sagittalebene verläuft.

4. Vorrichtung nach einem der Ansprüche 1 bis 3, dadurch gekennzeichnet, daß die horizontale Führung (18) von einer Schiene (40) gebildet ist und daß an den Enden der Schiene (40) senkrecht dazu verlaufende Arme (44, 46) angebracht sind, die mit länglichen Halteprofilen (52, 60 bzw. 54, 62) am Gehäuse des Computertomographen zusammenwirken.

5. Vorrichtung nach Anspruch 4, dadurch gekennzeichnet, daß die Halteprofile eine Platte (52, 54) enthalten, auf denen eine Führungsleiste (60, 62) angeordnet ist, die sich nach unten und von der Platte fort rampenartig vergrößert und die Arme (44, 46) ein zu den Führungsleisten komplementäres Profil aufweisen sowie einen Rastbolzen (48, 50) oder dergleichen, der mit einer Rastbohrung (68, 70) der Führungsleisten (60, 62) zusammenwirkt.

6. Vorrichtung nach Anspruch 4 oder 5, dadurch gekennzeichnet, daß die Halteprofile am Gehäuse des Computertomographen justierbar angebracht sind.

## Claims

1. A targetting device for the straight lined introduction of an elongate instrument into a human body onto a target location determined with the help of a computer tomograph and marked by by a deposit, with
- a computer tomograph (CT) comprising a gantry (12), for producing transversal section pictures of the body in a transversal scanning plane,
- a rest (14) traversable along a guide horizontally in the longitudinal direction of the gantry for positioning the patient,
characterised in that
- the targetting device serves the straight-lined introduction of an elongate instrument in a certain transversal section plane, and that the targetting device comprises
- a horizontal guide (18) attached above the gantry (12) on the CT with a carriage (20) traversable along the horizontal guide, with locking means for locking the carriage (20) at any point of the horizontal guide (18),
- a laser device (22, 22') for projecting a target marking,
- a mounting device which pivotably mounts the laser device (22, 22') about a horizontal axis in a manner such that the laser beam is pivotable in a transversal plane which is located at a predetermined distance to the transversal scanning plane, and
- an angular scale (34) on the carriage (20) for displaying the pivoting angle of the laser device (22, 22').

2. A device according to claim 1, characterised in that the laser device (22) is formed as a unit for projecting a line cross.

3. A device according to claim 1, characterised in that the laser device is formed for projecting a cross, wherein a line laser is attached fixed in space and its irradiation plane runs in the transversal plane of the body, this transversal plane being located at a certain distance from the scanning plane of the CT, and a second line laser is pivotably mounted on the carriage about the horizontal axis and its irradiation plane runs in the sagittal plane.

4. A device according to one of the claims 1 to 3, characterised in that the horizontal guide (18) is formed by a rail (40) and that at the ends of the rail (40) there are attached arms (44, 46) perpendicularly thereto which cooperate with elongate retaining profiles (52, 60 and 54,62 respectively) on the housing of the computer tomograph.

5. A device accordng to claim 4, characterised in that the retaining profiles contain a plate (52, 54) on which there is arranged a guide strip (60, 62) which enlarges downwards and away from the plate in a ramp-like manner and the arms (44, 46) comprise a profile complementary to the guide strips as well as a latching bolt (48, 50) or likewise which cooperates with a latching bore (68, 70) of the guide strips (60, 62).

6. A device according to claim 4 or 5, characterised in that the retaining profile is adjustably mounted on the housing of the computer tomograph.

## Revendications

1. Dispositif de visée destiné à introduire en ligne droite un instrument long dans le corps humain vers une zone cible déterminée à l'aide d'un scanographe et repérée par une marque, comprenant
- un scanographe muni d'un portique (12) et destiné à produire des images de coupes transversales du corps dans un plan de balayage transversal,
- un lit (14), susceptible d'être déplacé horizontalement le long d'un système de guidage dans le sens longitudinal du portique, sur lequel est positionné le patient,
caractérisé en ce que
- le dispositif de visée destiné à introduire en ligne droite un instrument long dans le corps humain est utilisé dans un plan de coupe transversal déterminé et en ce que le dispositif de visée comporte
- un système de guidage (18) horizontal monté au-dessus du portique (12) du scanographe, muni d'un chariot (20) qui peut se déplacer le long du système de guidage horizontal et qui comporte des moyens de blocage destinés à immobiliser le chariot (20) en un point quelconque du système de guidage (18) horizontal,
- un dispositif laser (22, 22') destiné à projeter un repère cible,
- un dispositif de logement, qui permet de loger le dispositif laser (22, 22') de manière à pouvoir pivoter autour d'un axe horizontal, de telle sorte que le rayon laser peut pivoter dans un plan transversal, qui se situe à une distance prédéterminée du plan de balayage transversal, et
- une règle graduée (34) réalisée sur le chariot (20) et destinée à indiquer l'angle de pivotement du dispositif laser (22, 22').

2. Dispositif selon la revendication 1, caractérisé en ce que le dispositif laser (22) est conçu comme une unité destinée à projeter un rayon en forme de croix.

3. Dispositif selon la revendication 1, caractérisé en ce que le dispositif laser est conçu pour projeter un rayon en croix, un laser linéaire étant monté de manière immobile et son plan de rayonnement s'étendant dans le plan transversal du corps du patient, lequel plan est situé à une distance déterminée du plan de balayage du scanographe, et un deuxième laser linéaire étant monté de manière à pouvoir pivoter contre le chariot autour de l'axe horizontal et son plan de rayonnement s'étendant dans le plan sagittal.

4. Dispositif selon l'une quelconque des revendications 1 à 3, caractérisé en ce que le système de guidage horizontal (18) est formé par un rail (40) et en ce que les extrémités du rail (40) portent des bras (44, 46) qui s'avancent perpendiculairement en saillie par rapport au rail et qui agissent conjointement avec des profilés de maintien longitudinaux (52, 60 ou 54, 62) montés contre la cage du scanographe.

5. Dispositif selon la revendication 4, caractérisé en ce que les profilés de maintien comportent une plaque (52, 54), sur laquelle est disposée une baguette de guidage (60, 62), qui s'agrandit vers le bas et à partir de la plaque en formant un plan incliné, et les bras (44, 46) présentent un profil complémentaire aux baguettes de guidage, ainsi que des boulons de serrage (48, 50) ou des éléments analogues qui agissent conjointement avec un alésage (68, 70) réalisé dans les baguettes de guidage (60, 62).

6. Dispositif selon la revendication 4 ou 5, caractérisé en ce que les profilés de maintien sont montés de manière à être ajustés contre la cage du scanographe.
